# FASCICULE DE BREVET EUROPEEN

(11) **EP 2 398 626 B1**
(45) Date de publication et mention de la délivrance du brevet: **14.08.2013**
(21) Numéro de dépôt: 10704584.1
(22) Date de dépôt: 26.01.2010
(51) Int. Cl.: B25B 15/00, A61B 17/88, A61C 8/00, A61B 17/86

(54) **TOURNEVIS PENTAGONAL, POUR VISSAGE DE VIS À EMPREINTE PENTAGONALE, EN PARTICULIER DE VIS ORTHOPÉDIQUES**
FÜNFECKIGER SCHRAUBENZIEHER ZUM SCHRAUBEN VON FÜNFECKIGEN PRÄGUNGSSCHRAUBEN, INSBESONDERE ORTHOPÄDISCHEN SCHRAUBEN
PENTAGONAL SCREWDRIVER, FOR SCREWING OF SCREWS WITH PENTAGONAL RECESS, IN PARTICULAR ORTHOPEDIC SCREWS

(30) Priorité: 23.02.2009 FR 0900808
(43) Date de publication de la demande: 28.12.2011
(73) Titulaire: Biotech International (Société par Actions Simplifiée), 13300 Salon de Provence (FR)
(72) Inventeur: IMPELLIZZERI, Frédéric, F-13300 Salon de Provence (FR)
(74) Mandataire: Marek, Pierre
(86) Numéro de dépôt international: PCT/FR2010/000064
(87) Numéro de publication internationale: WO 2010/094847

(56) Documents cités:
- DE-A1-102006 043 784
- FR-A- 2 201 645
- JP-A- 10 146 772

## Description

La présente invention concerne un tournevis pentagonal selon le préambule de la revendication 1 et un système de visserie comportant un tel tournevis. Un tel tournevis est connu du document FR 2201645 A.

Les vis à empreinte polygonale sont bien connues et largement utilisées comme éléments de fixation ou d'assemblage, en particulier les vis à empreinte hexagonale. De telles vis sont utilisées dans des industries diverses et notamment en chirurgie orthopédique pour réunir des fragments d'os, afin de réaliser la réduction des fractures osseuses par ostéosynthèse. L'entraînement en rotation de ces vis est obtenu au moyen de tournevis ou de clés présentant une extrémité de manoeuvre de section complémentaire de celle de l'empreinte des vis.

Le principal inconvénient résultant de l'utilisation de vis à empreinte hexagonale et de tournevis hexagonaux est que, les angles au sommet d'un hexagone valant 120°, le phénomène de ripage des tournevis dans l'empreinte se manifeste plus facilement. En effet, plus il y a de faces et plus les angles sont grands, plus la forme géométrique tend vers le cercle dans lequel elle est inscrite. Il y a ainsi peu de matière à déformer par le tournevis pour gauchir l'empreinte de la tête de vis. Le phénomène de ripage se trouve amplifié par le fait que, pour pouvoir introduire le tournevis dans l'empreinte d'une vis, il faut nécessairement qu'un jeu soit ménagé entre les faces en regard dudit tournevis et de ladite empreinte.

De plus, l'effort est transmis par l'intermédiaire des six arêtes verticales du tournevis qui s'usent donc et déforment l'empreinte de la vis, ce qui peut avoir comme conséquence de ne plus pouvoir retirer la vis de la matière osseuse dans laquelle elle a été vissée.

La solution évidente à ces inconvénients consisterait à réaliser des vis à empreinte comportant un nombre de pan inférieur à 6. En effet, en réduisant la valeur des angles au sommet du polygone (pentagone, carré, triangle), on diminue ou supprime le ripage du tournevis conformé de manière complémentaire.

Toutefois, cette solution n'est pas applicable à la plupart des vis utilisées en chirurgie orthopédique, en raison du fait que les têtes de ces vis présentent, dans leur portion proximale, un diamètre très réduit, généralement de l'ordre de 2 mm à 3 mm, d'autant que ces têtes présentent, le plus souvent, une forme conique et que leur diamètre diminue donc progressivement.

Si l'on considère, par exemple, un système de visserie utilisant des vis à empreinte carrée et un tournevis conformé de manière complémentaire, les angles au sommet d'un carré valant 90°, il y a une bonne transmission de l'effort. Cependant, pour une section équivalente à celle d'un hexagone, le carré est inscrit dans un cercle de plus grand diamètre, et ce d'autant plus lorsque la section de celui-ci est réduite de la valeur de la section de la broche guide. Ainsi, pour pouvoir utiliser une vis à empreinte carrée, il faut une tête de vis de diamètre beaucoup plus important que celui des vis munies d'une empreinte hexagonale. Or, en chirurgie orthopédique, les têtes de vis mesurent, comme indiqué précédemment, de 2 à 3 mm. Elles ne peuvent donc porter que de très petites empreintes carrées, dont la distance entre les 2 faces opposées serait voisine du diamètre de perçage recevant la broche guide, et dans ce cas, compte tenu de la très faible section résultante, le tournevis et/ou la tête de vis ne résisteraient pas à l'effet de la force de torsion.

Les empreintes triangulaires qui transmettraient encore mieux l'effort du fait de leurs angles de 60° présenteraient le même inconvénient que les empreintes carrées, dans l'application aux vis orthopédiques.

Un bon compromis permettant d'avoir des angles les plus petits possibles, afin de bien transmettre le couple d'effort, et une section d'empreinte et d'extrémité du tournevis la plus grande possible, pour que ce dernier ne casse pas sous l'effet de torsion et que l'empreinte de la tête de vis ne soit pas déformée serait donc l'utilisation de vis à empreinte pentagonale et d'un tournevis pentagonal.

Il a déjà été proposé des écrous à empreinte pentagonale et des clés d'entraînement pentagonales.

Par exemple, le document JP10146772 A décrit une clé Allen de section transversale de forme pentagonale et une vis de fixation à empreinte pentagonale qui permettent de réduire l'abrasion de l'extrémité distale de la clé qui est insérée dans l'empreinte de l'écrou.

Le document GB2109079 A décrit des écrous de fixation présentant une queue munie d'un alésage axial fileté et une tête conique munie d'un trou hexagonal ou pentagonal, essentiellement utilisés pour assurer la fixation de boulons en U pour des structures métalliques de jeux pour enfants, assurant une fixation sécurisée et permettant d'éviter les arêtes vives, et pouvant être utilisés avec des clés standard.

Cependant, l'utilisation, en soi, de systèmes de visserie comportant des clés ou tournevis pentagonaux et des vis à empreinte pentagonale ne présente pas un progrès très significatif par rapport aux systèmes utilisant des tournevis et des vis à empreinte hexagonale, de sorte que l'on ne connait pas de tels systèmes appliqués concrètement dans l'industrie et qu'ils sont inappliqués dans le domaine de la chirurgie orthopédique.

De plus, dans de tels systèmes de visserie, la génératrice qui transmet l'effort est constituée des cinq arêtes verticales. Il existe donc toujours un effet de ripage bien qu'il soit moins important que pour les tournevis hexagonaux du fait qu'il y a plus de matière à déformer.

Un but de la présente invention est de remédier aux inconvénients sus mentionnés en apportant une solution fiable et précise pour permettre une pose de vis plus performante et plus sécurisée, en particulier en chirurgie orthopédique.

Selon l'invention, ce but est atteint grâce à un tournevis pentagonal selon la revendication 1.

Le tournevis selon l'invention procure plusieurs avantages intéressants. Il permet notamment :
- de rattraper le jeu qui existe entre un tournevis et l'empreinte d'une tête de vis ;
- d'éviter le ripage, en appliquant fermement les faces du tournevis opposées au pan incliné contre les faces correspondantes de l'empreinte de la tête de vis ;
- de transmettre l'effort par l'intermédiaire d'une arête, de deux faces et d'un angle ; et
- d'avoir un effet préhenseur qui permet de maintenir la vis sur le tournevis.

Selon un mode d'exécution avantageux, la face pentée du tournevis présente une inclinaison comprise entre 2° et 3°.

Selon un mode d'exécution préféré, le pan incliné présente une inclinaison de 2° pour l'utilisation avec des vis comportant une tête de diamètre de 2 mm ou inférieur à 2 mm et une inclinaison de 3° pour l'utilisation avec des vis comportant une tête de diamètre supérieur à 2 mm.

Selon une autre disposition caractéristique, le tournevis selon l'invention présente un alésage axial permettant le passage d'une broche de perforation utilisée lors de l'exécution de certaines interventions de chirurgie orthopédique.

Selon un mode de réalisation préféré, le tournevis est en tout matériau dit chirurgical présentant la dureté nécessaire.

Selon un mode de réalisation avantageux, le tournevis selon l'invention est en acier inoxydable ayant subit un traitement thermique dans le but d'augmenter sa dureté.

Les buts, caractéristiques et avantages ci-dessus, et d'autres encore, ressortiront mieux de la description qui suit et des dessins annexés dans lesquels :

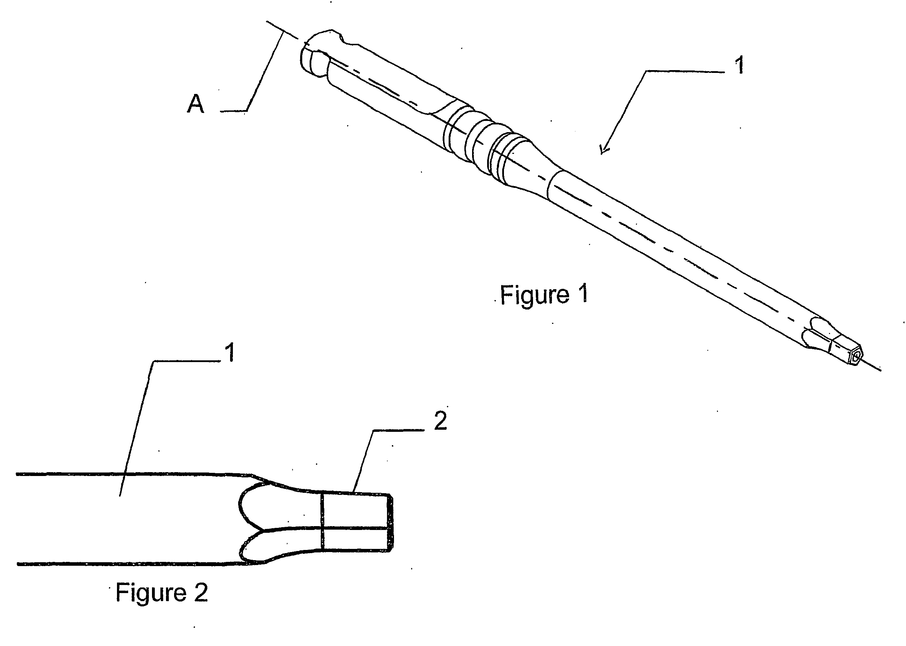

La figure 1 est une vue en perspective d'un exemple de réalisation du tournevis pentagonal selon l'invention.
La figure 2 est une vue de face et à plus grande échelle de la partie distale du tournevis pentagonal illustrant le pan incliné.
La figure 3 est une vue de dessous et à échelle agrandie de la partie distale du tournevis.
La figure 4 est une vue de détail, en coupe et à plus grande échelle, d'un tournevis pentagonal en cours d'introduction dans une tête de vis à empreinte pentagonale.
La figure 5 est une vue analogue à la figure 4 et montrant le tournevis totalement introduit dans une tête de vis à empreinte pentagonale.
La figure 6 est une vue de dessus, en coupe selon la ligne 6-6 de la figure 5 et représentant les arêtes ou les surfaces qui transmettent le couple de vissage.

On se réfère auxdits dessins pour décrire un exemple intéressant, quoique nullement limitatif, du tournevis pentagonal selon l'invention.

Le tournevis pentagonal 1 selon l'invention comporte une extrémité distale d'entrainement à cinq pans dont un pan 2 est incliné par rapport à l'axe longitudinal A du tournevis et en direction dudit axe.

Selon un mode d'exécution avantageux, ladite face inclinée 2 présente une inclinaison comprise entre 2° et 3°.

Préférentiellement, ladite inclinaison vaut 2° pour l'utilisation avec des vis comportant une tête de diamètre de 2 mm ou inférieur à 2 mm et 3° pour l'utilisation avec des vis comportant une tête de diamètre supérieur à 2 mm.

Selon l'exemple illustré, le tournevis pentagonal 1 présente un alésage axial 5 permettant le passage d'une broche guide.

Selon un mode de réalisation avantageux, le tournevis 1 est en tout matériau dit chirurgical présentant la dureté nécessaire, par exemple en acier inoxydable.

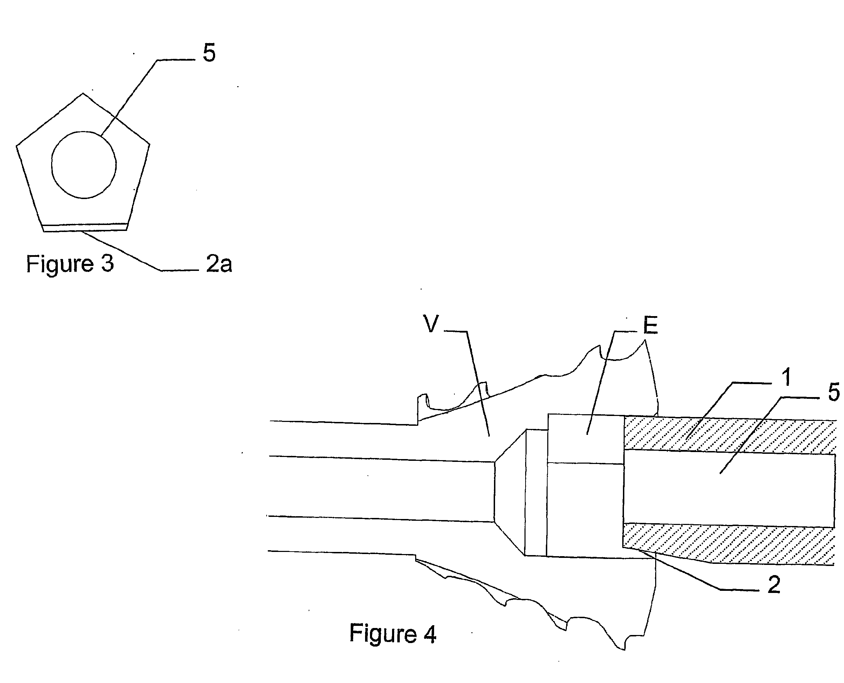

Selon l'exemple illustré à la figure 4, le pan ou face inclinée 2 permet, dans un premier temps, de ménager un jeu qui autorise l'introduction du tournevis 1 dans l'empreinte pentagonale ou à cinq pans creux E de la tête de vis V.

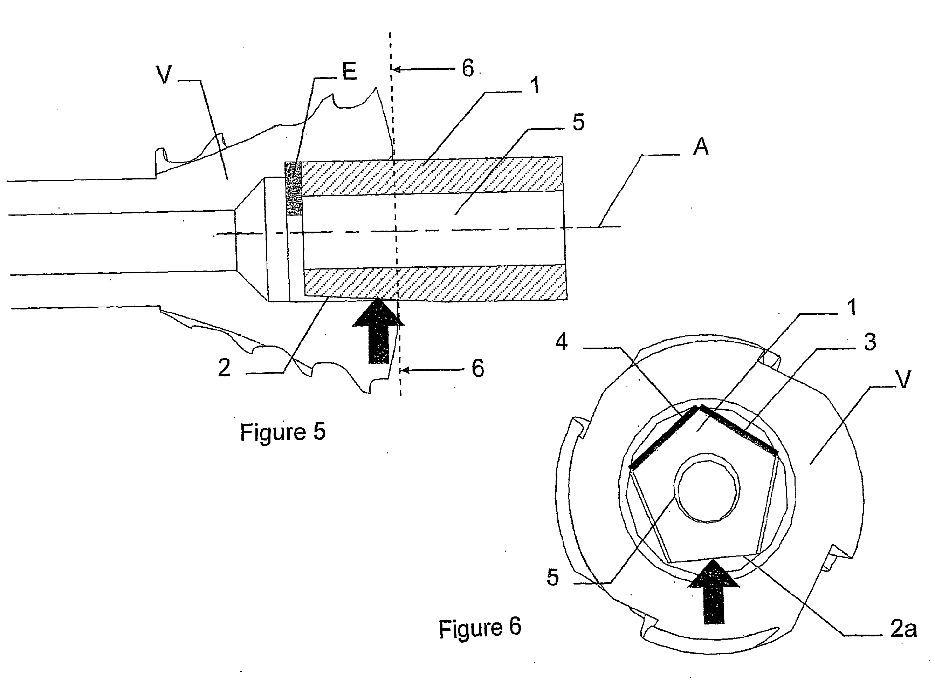

Au fur et à mesure de l'introduction du tournevis 1 dans l'empreinte E de la tête de vis, le jeu diminue et disparaît totalement, permettant ainsi au tournevis d'épouser parfaitement la forme de l'empreinte E de la vis V (figure 5).

De plus, lorsque le tournevis 1 est complètement introduit dans l'empreinte E de la tête de vis V, la face inclinée 2 permet de plaquer fermement les deux faces ou pans 3 et 4, opposés audit pan incliné 2, contre les pans creux correspondants de l'empreinte de la vis, comme le montre la flèche des figures 5 et 6.

Le tournevis 1 se trouve alors immobilisé dans la tête de vis V, et le pan incliné génère un effet de préhension.

Ainsi, l'effort appliqué sur le tournevis 1 pour visser ou dévisser une vis orthopédique est transmis à ladite vis par l'intermédiaire de l'arête 2a du pan incliné 2, par les faces opposées 3 et 4 audit pan incliné 2 et par l'angle compris entre lesdites faces 3 et 4.

Le pentagone du tournevis 1 étant convexe et régulier, ses angles intérieurs valent 108° ce qui permet une meilleure transmission de l'effort que celle obtenue aux moyens des systèmes de visserie utilisant des vis à six pans creux et des tournevis dont les angles ont une valeur de 120°.

L'utilisation de cette forme géométrique est encore remarquable en ce que la surface du cercle dans lequel le pentagone est inscrit et qui n'est pas occupée par le pentagone est plus importante que pour un hexagone.

Ainsi, on élimine toute manifestation du phénomène de ripage conduisant à une déformation de la matière de la tête de vis orthopédique V.

L'invention concerne également un système de visserie comprenant des vis à empreinte pentagonale et un tournevis selon la revendication 1.

## Revendications

1. Tournevis pentagonal, pour vissage de vis à empreinte pentagonale, en particulier de vis orthopédiques, qui possède un pan incliné (2) par rapport et en direction de l'axe longitudinal (A) dudit tournevis (1), **caractérisé en ce que** les quatre autres pans du tournevis sont droits et parallèles audit axe longitudinal.

2. Tournevis pentagonal selon la revendication 1, **caractérisé en ce que** l'inclinaison du pan (2) est comprise entre 2° et 3°.

3. Tournevis pentagonal selon l'une quelconque des revendications 1 ou 2, **caractérisé en ce que** le pan (2) est incliné de 2° pour l'utilisation avec des vis comportant une tête de diamètre de 2 mm ou inférieur à 2 mm.

4. Tournevis pentagonal selon l'une quelconque des revendications 1 ou 2, **caractérisé en ce que** le pan (2) est incliné de 3° pour l'utilisation avec des vis comportant une tête de diamètre supérieur à 2 mm.

5. Tournevis pentagonal selon l'une quelconque des revendications 1 à .3, **caractérisé en ce qu'**il est muni d'un alésage axial (3).

6. Tournevis pentagonal selon l'une quelconque des revendications 1 à 4, **caractérisé en ce qu'**il est en tout matériau dit chirurgical présentant la dureté nécessaire.

7. Tournevis pentagonal selon l'une quelconque des revendications 1 à 5, **caractérisé en ce qu'**il est en acier inoxydable.

8. Tournevis pentagonal selon l'une quelconque des revendications 1 à 5, **caractérisé en ce qu'**il est en acier inoxydable ayant subit un traitement thermique dans le but d'augmenter sa dureté.

9. Système de visserie comprenant un tournevis selon l'une quelconque des revendications 1 à 8 et des vis à empreinte pentagonale.

## Patentansprüche

1. Fünfeck-Schraubendreher zum Einschrauben von Schrauben mit fünfeckigen Ausnehmungen, insbesondere von orthopädischen Schrauben, der eine geneigte Fläche (2) relativ zu und in Richtung der Längsachse (A) des Schraubendrehers (1) besitzt **dadurch gekennzeichnet, dass** die vier anderen Flächen des Schraubendrehers gerade und parallel zu der Längsachse sind.

2. Fünfeck-Schraubendreher nach Anspruch 1, **dadurch gekennzeichnet, dass** die Neigung der Fläche (2) zwischen 2° und 3° ist.

3. Fünfeck-Schraubendreher nach einem der Ansprüche 1 oder 2, **dadurch gekennzeichnet, dass** die Fläche (2) um 2° geneigt ist, zur Verwendung mit Schrauben mit einem Kopfdurchmesser von 2 mm oder weniger als 2 mm.

4. Fünfeck-Schraubendreher nach einem der Ansprüche 1 oder 2, **dadurch gekennzeichnet, dass** die Fläche (2) um 3° geneigt ist, zur Verwendung mit Schrauben einen Kopfdurchmesser größer als 2 mm.

5. Fünfeck-Schraubendreher nach einem der Ansprüche 1 bis 3, **dadurch gekennzeichnet, dass** er mit einer axialen Bohrung (3) versehen ist.

6. Fünfeck-Schraubendreher nach einem der Ansprüche 1 bis 4, **dadurch gekennzeichnet, dass** er aus chirurgischem Material mit der erforderlichen Härte ausgebildet ist.

7. Fünfeck-Schraubendreher nach einem der Ansprüche 1 bis 5, **dadurch gekennzeichnet, dass** er aus rostfreiem Stahl hergestellt ist.

8. Fünfeck-Schraubendreher nach einem der Ansprüche 1 bis 5, **dadurch gekennzeichnet, dass** es aus rostfreiem Stahl ausgebildet ist, der einer Wärmebehandlung unterzogen wurde, um die Härte zu erhöhen.

9. Verschraubungs-System umfassend einen Schraubendreher nach einem der Ansprüche 1 bis 8 und Schrauben mit fünfeckigen Ausnehmungen.

## Claims

1. A pentagonal screwdriver for screwing screws with a pentagonal recess, in particular orthopaedic screws, which has a flat (2) which is inclined relative to and in the direction of the longitudinal axis (A) of said screwdriver (1), **characterised in that** the other four flats of the screwdriver are straight and parallel to said longitudinal axis.

2. A pentagonal screwdriver according to claim 1 **characterised in that** the inclination of the flat (2) is between 2° and 3°.

3. A pentagonal screwdriver according to either one of claims 1 and 2 **characterised in that** the flat (2) is inclined at 2° for use with screws comprising a head of a diameter of 2 mm or less than 2 mm.

4. A pentagonal screwdriver according to either one of claims 1 and 2 **characterised in that** the flat (2) is inclined at 3° for use with screws comprising a head of a diameter of greater than 2 mm.

5. A pentagonal screwdriver according to any one of claims 1 to 3 **characterised in that** it is provided with an axial bore (3).

6. A pentagonal screwdriver according to any one of claims 1 to 4 **characterised in that** it is of any material referred to as surgical material of the necessary hardness.

7. A pentagonal screwdriver according to any one of claims 1 to 5 **characterised in that** it is of stainless steel.

8. A pentagonal screwdriver according to any one of claims 1 to 5 **characterised in that** it is of stainless steel which has been subjected to a heat treatment to increase its hardness.

9. A screwing system comprising a screwdriver according to any one of claims 1 to 8 and screws with a pentagonal recess.
